# EUROPEAN PATENT SPECIFICATION

(11) **EP 3 789 086 B1**
(45) Date of publication and mention of the grant of the patent: **24.12.2025**
(21) Application number: 19792520.9
(22) Date of filing: 31.05.2019
(51) Int. Cl.: A61N 5/10, A61N 5/00

(54) **RADIOTHERAPY DEVICE**
STRAHLENTHERAPIEVORRICHTUNG
DISPOSITIF DE RADIOTHÉRAPIE

(30) Priority: 28.04.2018 CN 201810404591
(43) Date of publication of application: 10.03.2021
(73) Proprietor: Shenzhen Mingjie Medical Technology Co. Ltd., Shenzhen, Guangdong 518048 (CN)
(72) Inventor: GAI, Wei, Shenzhen, Guangdong 518048 (CN)
(74) Representative: Boult Wade Tennant LLP
(86) International application number: PCT/CN2019/089581
(87) International publication number: WO 2019/206342

(56) References cited:
- CN-A- 1 537 653
- CN-A- 105 662 578
- CN-A- 105 662 578
- US-A- 5 317 164
- US-A- 5 585 643
- US-A1- 2016 121 137

## Description

### FIELD

Embodiments of the present disclosure generally relate to a radiotherapy apparatus.

### BACKGROUND

Tumour is a disease endangering human life. A tumor therapy method mainly depends on surgery, chemotherapy and radiotherapy. However, the surgery, the chemotherapy and the radiotherapy all cause deep pain to a patient. For example, the surgery not only has high treatment cost, but also has high risk and great therapy difficulty. In addition, the patient is required to go through a long period of wound healing and convalescence after surgery. X-rays have strong penetrability. In therapy, the X-rays are irradiated from outside the body of the patient and pass through the body. Therefore, a lot of energy is accumulated in normal tissues in front of and behind a tumour lesion site, which causes harm to the body.

In US patent Number 5,585,643, a device for directing electron radiation to subcutaneous cells is provided. The device includes a hollow needle having a proximal and a distal end for respectively directing the electron beam therethrough. The proximal end of the needle is adapted for attachment to the source of the electron beam. A scattering target for the electron beam is also provided. The scattering target is adapted for positioning at the distal end of the hollow needle whereby the distal end of the hollow needle is capable of insertion directly to the subcutaneous cells for radiation treatment.

In Chinese Patent Publication No. CN105662578, a puncture needle propelling mechanical arm is provided. The puncture needle propelling mechanical arm includes a mechanical arm body, a propulsion device and an ablation needle. The mechanical arm body includes: a bottom flange, a top flange, and a six-axis mechanical arm arranged between the bottom flange and the top flange. The propulsion device includes a frame provided with a linear guide rail and a movable slide table. The ablation needle is provided at the bottom of the movable slide table by a ablation needle clamp. Document US 5 317 164 A discloses a radiotherapy apparatus wherein an electron beam is administered to the target through a needle. The needle is insertable into the target. Position and orientation of the needle are adjustable.

### SUMMARY

An object of embodiments of the present disclosure is to provide a radiotherapy apparatus to accurately destroy diseased tissues in human bodies or animal bodies without causing harm to normal tissues, thereby reducing harm to human bodies or animal bodies.

The invention is defined in the claims. Other embodiments, methods, examples, items etc. are not a part of the invention and are provided for illustrative purposes only.

### BRIEF DESCRIPTION OF THE DRAWINGS

One or more embodiments are illustratively described in conjunction with the drawings corresponding to the embodiments. The illustratively descriptions are not used to limit the embodiments. In the drawings, components with the same reference numeral sign are similar. The drawings do not limit ratio of component dimensions unless otherwise stated.
Figure 1 is a schematic structural diagram of a radiotherapy apparatus according to a first embodiment of the present disclosure;
Figure 2 is a schematic structural diagram showing an electron beam emitting device and a beam needle drawn into a tumor lesion site according to the first embodiment of the present disclosure;
Figure 3 are diagrams showing parameters of simulated high brightness electron beams generated in a process of emitting electron beams according to the first embodiment of the present disclosure;
Figure 4 is a schematic structural diagram of a radiotherapy apparatus according to a second embodiment of the present disclosure;
Figure 5 is a schematic structural diagram of a hand-eye system of the radiotherapy apparatus according to the second embodiment of the present disclosure;
Figure 6 is a flowchart of a therapy method using the radiotherapy apparatus according to the second embodiment of the present disclosure;
Figure 7 is a schematic structural diagram of a radiotherapy apparatus according to a third embodiment of the present disclosure;
Figure 8 is a schematic diagram showing a partial structure of the radiotherapy apparatus according to the third embodiment of the present disclosure;
Figure 9 is a flowchart of a therapy method using the radiotherapy apparatus according to the third embodiment of the present disclosure;
Figure 10 is a schematic structural diagram showing a motion device arranged in a fixed mount according to a fourth embodiment of the present disclosure;
Figure 11 is a schematic structural diagram of a radiotherapy apparatus without a travelling crane according to a fifth embodiment of the present disclosure;
Figure 12 is a schematic structural diagram of a radiotherapy apparatus with a travelling crane according to a sixth embodiment of the present disclosure;
Figure 13 is a diagram showing circuit modules of the radiotherapy apparatus according to the fifth embodiment;
Figure 14 is a diagram showing circuit modules of the radiotherapy apparatus according to the sixth embodiment;
Figure 15 is a schematic structural diagram of a radiotherapy apparatus according to a seventh embodiment of the present disclosure;
Figure 16 is a flowchart of a therapy method using anyone of the radiotherapy apparatuses according to the first, the fourth and the fifth embodiments of the present disclosure;
Figure 17 is a flowchart of a therapy method using anyone of the radiotherapy apparatuses according to the sixth and the seventh embodiments of the present disclosure; and
Figure 18 is a flowchart of another therapy method using anyone of the radiotherapy apparatuses according to the sixth and the seventh embodiments of the present disclosure.

### DETAILED DESCRIPTION OF EMBODIMENTS

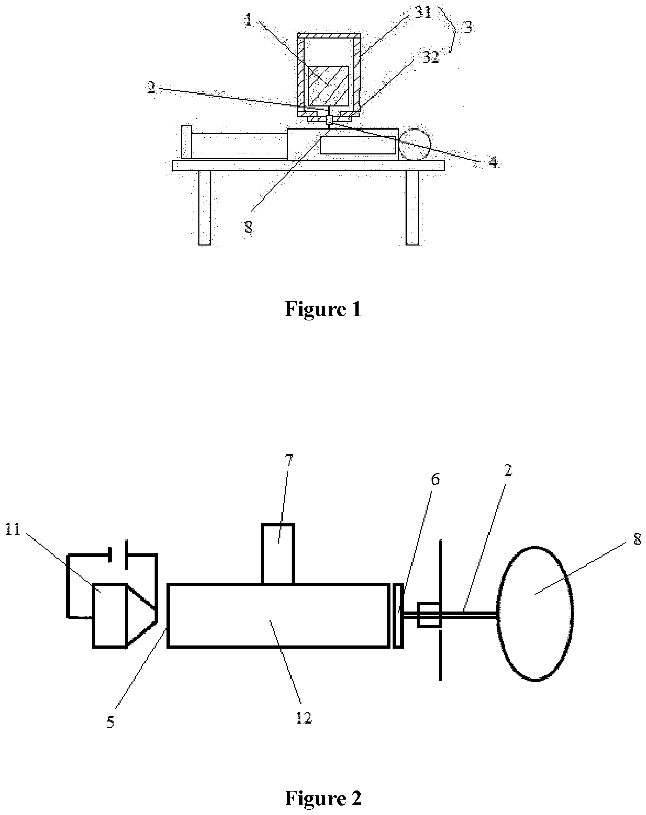

In order to make the objective, technical solutions and advantages of the embodiments of the present disclosure clear, the embodiments of the present disclosure are described in detail below in conjunction with the drawings. Those skilled in the art can understand that many technical details are described in the embodiments of the present disclosure to cause the disclosure to be understood well by readers. However, claimed technical solutions of the present disclosure can be implemented without the technical details and various variations and modifications made based on the following embodiments. A first embodiment of the present disclosure relates to a radiotherapy apparatus. The invention is defined in the claims. Methods of therapy and surgery described below, are not a part of the invention. As shown in Figure 1 and Figure 2, the radiotherapy apparatus includes an electron beam emitting device 1, a beam needle 2 and a motion device 3. The motion device 3 is configured to drive the beam needle 2 to move in a straight line to cause the beam needle 2 to be drawn into a tumour lesion site 8 in a body through a trocar 4 prearranged on the body. The electron beam emitting device 1 is connected to the beam needle 2 and may be configured to emit an electron beam after being delivered to an operation position by the motion device 3. The electron beam is shot at the tumour lesion site 8 in the body through the beam needle 2 so as to ablate the tumour. In addition, after the electron beam is shot at the tumour lesion site 8 through the beam needle 2, the beam needle 2 is drawn out of the body through the trocar 4 under the drive of the motion device 3.

As can be seen from the above, the whole radiotherapy apparatus includes the electron beam emitting device 1, the beam needle 2 and the motion device 3. In practice, the motion device 3 may drive the beam needle 2 to move in a straight line so as to draw the beam needle 2 into a diseased tissue or a lesion site in the body such as a tumour lesion site through the trocar 4 prearranged on the body. The electron beam emitting device 1 emits an electron beam and the electron beam is shot at the tumour lesion site through the beam needle 2 to ablate the tumour. In addition, after the electron beam is shot at the tumour lesion site, the beam needle 2 may be drawn out of the body through the trocar 4 under the drive of the motion device 3. In this way, the electron beam is accurately shot at the tumour moving following an organ, which avoids shot deviation of the electron beam due to inaccurate positioning of the beam needle. Furthermore, the electron beam has a weak penetrability. Therefore, not too much energy is accumulated in normal tissues in front of and behind the tumour lesion site 8, which avoids harm to normal tissues when the electron beam ablates the tumour, thereby reducing harm to the body and reducing pain the patient feels in therapy.

In the embodiment, as shown in Figure 2, the electron beam emitting device 1 includes an electron gun 11 and an accelerating tube 12. The accelerating tube 12 may be an X-band accelerating tube or a C-band accelerating tube. A center frequency of the X-band accelerating tube is 9.3GHz or 11.424 GHz. A center frequency of the C-band accelerating tube is 5712MHz. An end of the accelerating tube 12 is an inlet 5 for the electron beam and the inlet 5 is connected to the electron gun 11. The other end of the accelerating tube 12 is an outlet 6 for the electron beam and the outlet 6 is connected to the beam needle 2. The electron gun 11 emits an electron beam and the electron beam enters the accelerating tube 12 via the inlet 5 of the accelerating tube 12. The accelerating tube 12 accelerates the electron beam entering the accelerating tube 12 and the accelerated electron beam enters the beam needle 2 via the outlet 6. The accelerating tube 12 is further provided with a microwave inlet 7 configured to input microwave to the accelerating tube 12. When the electron gun 11 emits an electron beam, microwave may be inputted into the accelerating tube 12 via the microwave inlet 7 simultaneously, thereby accelerating the electron beam and increasing energy of the electron beam.

A process of ablating a tumour with the radiotherapy apparatus according to the embodiment is described as following.

### 1. Generation of a high brightness electron beam

In the embodiment, the high brightness electron beam refers to as an electron beam with normalized emittance no greater than 10 millimeter milliradian and energy ranging from 1MeV to 100MeV. Only an electron beam with a high enough brightness can be transmitted without losses in the beam needle 2 with a diameter no greater than 2mm and reach the tumor lesion site successfully. The generation of the high brightness electron beam includes emission of the electron beam and acceleration of the electron beam. Both the emission and the acceleration of the electron beam are important for shooting the electron beam at the tumor lesion site.

### (1) The emission of the high brightness electron beam

As a common device for generating a high brightness electron beam in the field of accelerator, the electron gun 11 has a high accelerating gradient, which restrains space charge effect effectively. A cathode of the electron gun 11 may be a photocathode, a thermionic cathode or a field emission cathode, which depends on an emission mode. For the photocathode, laser activates electrons in the cathode to cause the activated electrons to escape from the cathode. Electron beams generated in this mode have advantages of high peak flow intensity and low emittance. The technology for generating electron beams in this mode is mature but is not applied in the medical filed at present. Dozens of such electron guns 11 have been successfully developed in the world, including China, and have been successfully applied in various scientific devices. For the thermionic cathode, the thermionic cathode is heated to emit electrons. Electron beams generated in this mode have advantages of high average flow intensity and low emittance. The thermionic cathode has been widely applied in the world. For the field emission cathode, electrons are drawn out of the cathode under an action of a strong electric field on a surface of the cathode. Electron beams generated in this mode have advantages of low emittance and high average flow intensity. However, the field emission cathode is demanding on materials.

In summary, conventional technologies can fully meet present demands. It is proved through current experiments that an electron gun 11 with anyone of the above three types of cathode may be used to generate the high brightness electron beam meeting requirements in the embodiment. In the near future, with the deep research on the electron guns 11 respectively with the three types of cathode, a best solution for emitting electrons may be found by considering factors such as emittance of the electron beam, cathode lifetime and device cost.

### (2) The acceleration of the high brightness electron beam

The conventional accelerating tube 12 may generate an electron beam with energy ranging from 1MeV to 10MeV, which can basically meet requirements in the medical field. Parameters of the accelerating tube 12 may be optimized according to actual needs. In addition, common accelerating devices are supplied by mature suppliers. A technically mature solution (X-band solution) is only described herein.

Electron guns corresponding to many large accelerating tubes in the word can generate the high brightness electron beam such as a synchrotron radiation electron beam and a free electron laser. Relevant technologies are mature. The X-band solution is based on these existing methods for generating the high brightness electron beam. In the embodiment, the inventor designs a multicavity electron gun that can generate an electron beam with energy ranging from 2MeV to 10MeV and with an emittance less than 1 millimetre milliradian.

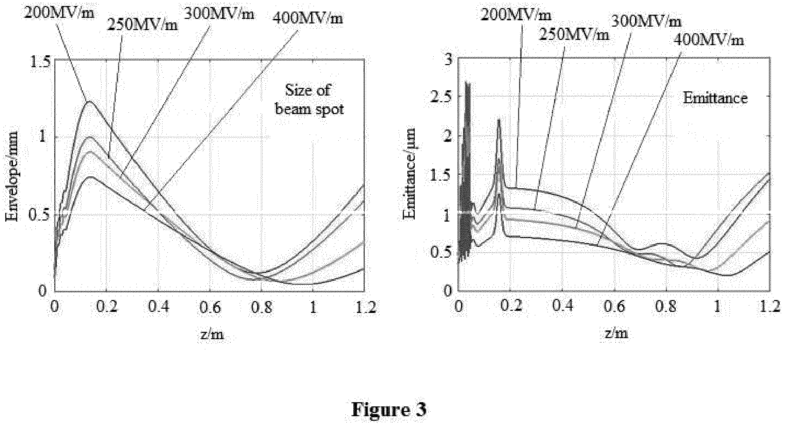

Figure 3 are diagrams showing parameters of beam spots and emittances of simulated high brightness electron beams generated by an X-band (11.424GHz) accelerating tube under an accelerating field ranging from 200MV to 400MV in a laboratory. For values of the parameters, one may refer to Table 1. The Figure indicates that a well-adjusted electron beam carrying 200pC of charges emitted by a radiofrequency gun with a photocathode can travel a long distance, for example, 10 centimeters to 40 centimeters in a small vacuum tube (that is, the beam needle 2). The accelerating tube 12 is an X-band accelerating tube. An outlet for electron beams of the X-band accelerating tube 12 is provided with a fence structure configured to partially intercept the high brightness electron beam. **In** addition, a tail end of the small vacuum tube (that is, the beam needle 2) may be connected to a thin layer of a material with low atomic number (for example, titanium or beryllium) configured to scatter the electron beam. The high brightness electron beam can be drawn out of the beam needle well by arranging a titanium window, a beryllium or a steel window at a tail end of the beam needle as an outlet window.

**Table 1 Parameters of an electron beam generated by the X-band accelerating tube**

| Accelerating gradient Ec [MV/m] | Beam emittance εₓ [µm] | Beam half width σₓ [mm] | Magnetic field intensity *B₀* [T] | Beam energy [MeV] |
|---|---|---|---|---|
| 200 | 0.42 | 0.12 | 0.36 | .7 |
| 250 | 0.32 | 0.08 | 0.47 | 5.8 |
| 300 | 0.26 | 0.07 | 0.55 | 7.0 |
| 400 | 0.20 | 0.04 | 9.3 | 40 |

The C-band (5712MHz) accelerating tube and the X-band (9.3GHz) accelerating tube may generate electronic beams with emittance the same as that of the electronic beam generated by the X-band (11.424GHz) accelerating tube. However, the C-band (5712MHz) accelerating tube and the X-band (9.3GHz) accelerating tube are different from the X-band (11.424GHz) accelerating tube in dimension and required power source. Relevant designs and calculations indicate that a C-band accelerating tube including three bunching cavities and ten light speed cavities may generate an electron beam with 6MeV of energy under 2MW of input power and 22MV/m of accelerating gradient. An X-band (9.3GHz) accelerating tube including four bunching cavities and seven light speed cavities may generate an electron beam with 4MeV of energy under 1.6MW of input power and 25MV/m of accelerating gradient.

The above data are obtained based on experiments and electron beams with other volume of energy may be acquired by adjusting parameters of conventional accelerators.

### 2. Transmission of the electron beam

It is easy to add a focusing magnet to the compact accelerator tube 12 due to a small lateral dimension of the compact accelerator tube 12. Therefore, an electron beam with an emittance less than 1 millimeter milliradian can be well focused and transmitted over a distance of 50 centimeter without loss. The dose range of the electron beam may be between 10Gy/min to 100Gy/min after acquiring enough energy through acceleration of the accelerating tube 12 (the X-band accelerating tube or the C-band accelerating tube). Finally, the electron beam is transmitted to the lesion site through vacuum with low loss.

The beam needle 2 is configured to transmit the high brightness electron beam through vacuum with low loss. A titanium window or beryllium window is used as an outlet window for the high luminance electron beam. The beam needle 2 has a millimeter scale diameter and a length ranging from 1 centimeter to 100 centimeter. An electron beam tube is required to have features of sufficient rigidity and non-magnetism. A vacuum butting part is required to be detachable and reused. The above technology may be implemented based on conventional high energy physics technology and medical apparatus and instruments technology, which are not repeated herein. The electron beam is transmitted from the beam needle 2 to the outside through vacuum with low loss or even no loss. Conventional titanium window technology and beryllium window technology basically meet requirements in the embodiment.

In addition, in transmission of the high brightness electron beam, sometimes it is also required to use a magnet configured to focus or direct the high brightness electron beam. For example, the accelerated high brightness electron beam enters the beam needle after being focused by the focusing magnet. Alternatively, the electron beam entering the beam needle 2 performs a direction adjustment under direction of the magnet.

### 3. Ablation capacity of the high brightness electron beam

In the above embodiments, the high brightness electron beam carrying 200pC of charges generated by the electron gun 11 using the X-band (11.424GHz) accelerator may enter a threadlike beam needle 2 (with a length ranging from 10 centimeter to 40 centimeter). The tail end of the beam needle 2 is connected to a beryllium tube or a titanium tube (which are also referred to as a beryllium window or a titanium window respectively) with low atomic number that can scatter a small amount of electron beams, so as to draw the high brightness electron beam towards the tumor. Generally, a compact accelerating tube 12 with 100Hz of repetition frequency that can generate an electron beam carrying 200pC of charges with 4MeV of energy may generate 80J of energy per second. Assuming that all energy is accumulated in a volume of 1 cubic centimeter, a dose about 80Gy/s may be reached, which certainly destroys almost any cancerous tissue. That is, theoretically, a diseased location may acquire a prescription dose of electron beams within a second, thereby completing tumor therapy.

### 4. Radiation protection

In the embodiment, the desired technical effect can be realized only using the compact accelerating tube 12, which causes the radiation protection to be very easy. Based on estimation, a lead plate with a thickness of 3 centimeter is enough to shield the radiation caused by the electrons, so that an expensive treatment room is not required. Based on monte carlo simulation performed on the whole apparatus, systematic analysis performed on dose distribution and experimental verification, it is indicated that radiation protection against the apparatus may be implemented in a partial shielding manner, which reduces a weight of a shielding material as much as possible, thereby keeping a weight of the whole apparatus under 20 kilogram. In addition, the whole apparatus is not required to operate in an isolation room. Medical care personnel can be protected from radiation without an additional shielding.

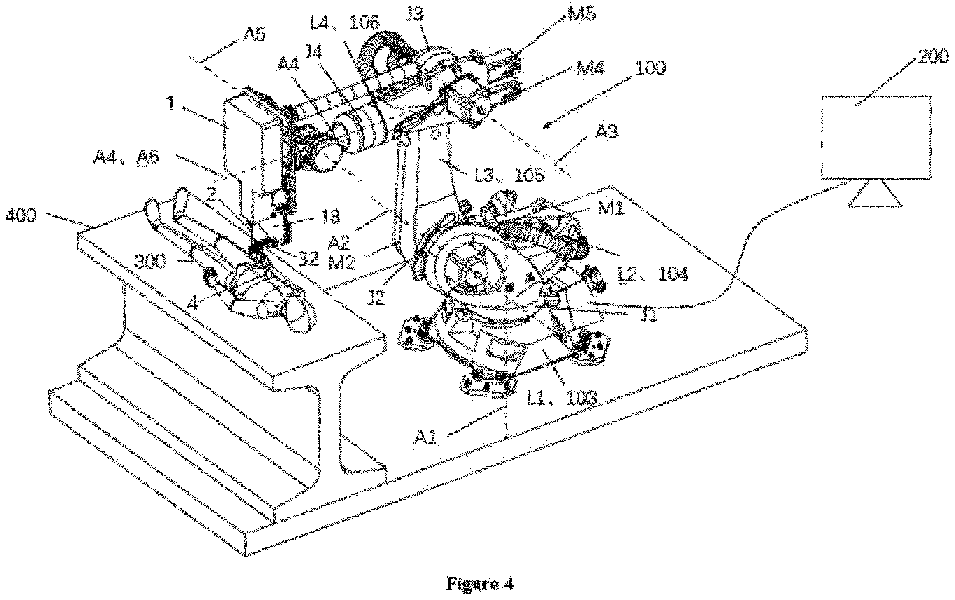

Figure 4 is a schematic structural diagram of a radiotherapy apparatus according to a second embodiment of the present disclosure. The radiotherapy apparatus includes a driving mechanism 100, a handpiece 1 and a control terminal 200. The driving mechanism 100 is configured to drive the handpiece 1 to move relative to a patient 300 or a trocar 4 so as to cause a beam needle 2 on the handpiece 1 to accurately reach a predetermined position and enter the body of the patient 300 following a predetermined path. The control terminal 200, for example, a computer, is connected to the driving mechanism 100 and the handpiece 1 in a wired manner or a wireless manner to deliver instructions to the driving mechanism 100 and the handpiece 1. The control terminal 200 receives real-time information such as position information, image information and dose information of the driving mechanism 100 and the handpiece 1 simultaneously.

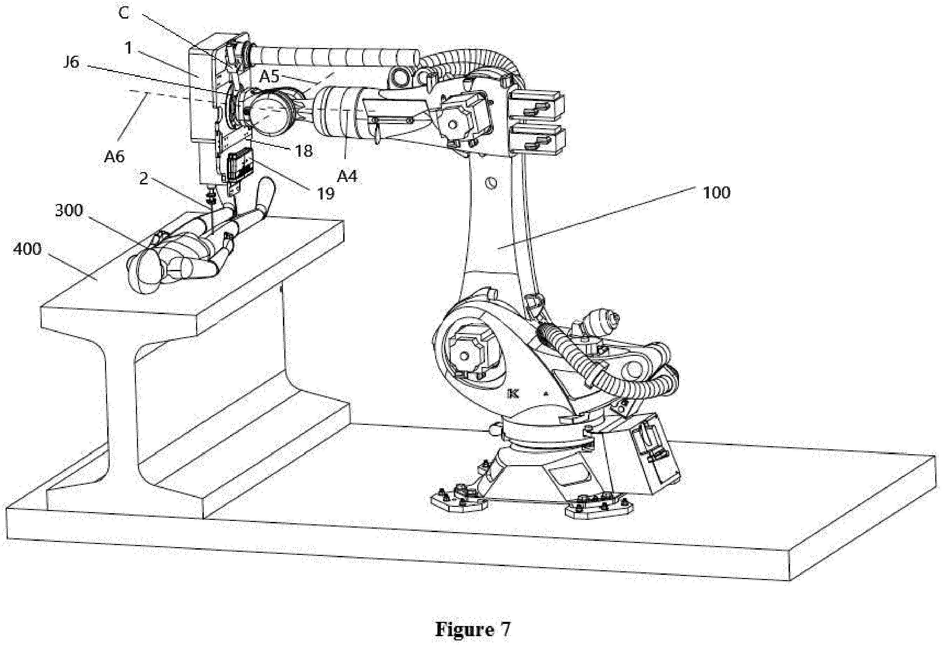

In the embodiment, the driving mechanism 100 is a six-axis articulated arm driving mechanism. The control terminal 200 of the driving mechanism 100 is designed or configured to execute a driving program. Joints J1 to J6 of a driving mechanism arm 102 may be automatically adjusted or move in a rational manner in an automated operation or a manual operation based on the driving program. For a structure of J6, one may refer to Figure 7. In order to implement the above functions, the control terminal 200 is connected to controllable electric motors M1 to M6. The electric motors are designed to adjust the joints J1 to J6 of the driving mechanism 100.

Elements L1 to L6 are related to a base 103 and a turntable 104 rotatably mounted surrounding an axis A1 extending vertically relative to the base 103. For L5 and L6, one may refer to Figure 7. Other elements of the driving mechanism arm 102 include a rocker arm 105 and a cantilever 106. A lower end of the rocker arm 105, that is, the joint J2 (the joint may also be referred to as a rocker arm support) of the rocker arm 105 is pivotally mounted on the turntable 104 surrounding a rotation axis A2 which is preferably horizontal.

On an upper end of the rocker arm 105, that is, on the first joint J3 of the rocker arm 105, the cantilever 106 is pivotally mounted surrounding an axis A3 which is also preferably horizontal. The joints J1 to J6 may be driven respectively by the electric motors M1 to M6 under a programming control of the control terminal 200. Therefore, an actuator may be arranged between each of the elements L1 to L6 and an electric motor assigned to the element.

The handpiece 1 is connected to the element L6 through a mounting structure. The mounting structure includes a baseplate 18, a sliding structure and a linkage structure C. The handpiece 1 is mounted on the baseplate 18 through the sliding structure so that the handpiece 1 and the baseplate 18 can slide up and down relative to each other. The baseplate 18 is in fixed communication with L6. J6 is connected to the handpiece 1 though the linkage structure C. In this way, J6 rotates so as to drive the handpiece 1 to move up and down. The baseplate 18 is preferably but is not limited to a metal plate. The baseplate 18 is provided with two sliding grooves that are parallel with each other. The sliding grooves are configured to cooperate with rails of the handpiece 1 to cause the handpiece 1 to move up and down in a direction perpendicular to the patient 300. The sliding grooves of the baseplate 18 and the rails of the handpiece 1 form the sliding structure. It can be understood that the rails may be arranged on the baseplate 18 and the sliding grooves may be arranged on the handpiece 1.

Here it should be noted that in practice, a defined handpiece may include a mounting structure. The mounting structure and the handpiece 1 are parts of the defined handpiece, which is because of a difference in name definition and there is no difference in essence.

The handpiece 1 is provided with an electron accelerator. The accelerator includes an electron emitting gun, an accelerating tube and a microwave generator. The electron emitting gun is configured to emit an electron beam. The common microwave generator includes a magnetron. The microwave generator is communicated with the accelerating tube to provide microwave for accelerating electrons. The beam needle 2 is in a fixed connection with the accelerating tube. The electron emitting gun emits an electron beam in response to instructions sent by the control terminal 200. The electron beam is accelerated by the accelerating tube and is finally shot out of the beam needle 2. The control terminal 200 may control a dose of the electron beam for treating the patient 300 by controlling a duration for which the electron emitting gun emits the electron beam.

In order to present a dose of the electron beam that have been entered the patient 300 in a real time manner, the radiotherapy apparatus may further be provided with a device for monitoring a dose in a real time manner. The device for monitoring a dose in a real time manner includes a sensor that can sense charges in the electron beam, for example, a current transformer. The sensor is arranged at a connection of the accelerating tube and the beam needle 2. The device for monitoring a dose in a real time manner is connected to the control terminal 200 and feeds back charge information/current information of the electron beam flowing through the device in a real time manner. The control terminal 200 acquires a real-time accumulated dose based on the charge information/current information. When the accumulated dose meets a predetermined condition, for example, the accumulated dose reaches a predetermined threshold value, the control terminal 200 sends a stopping command to cause the electron emitting gun to stop emitting electrons.

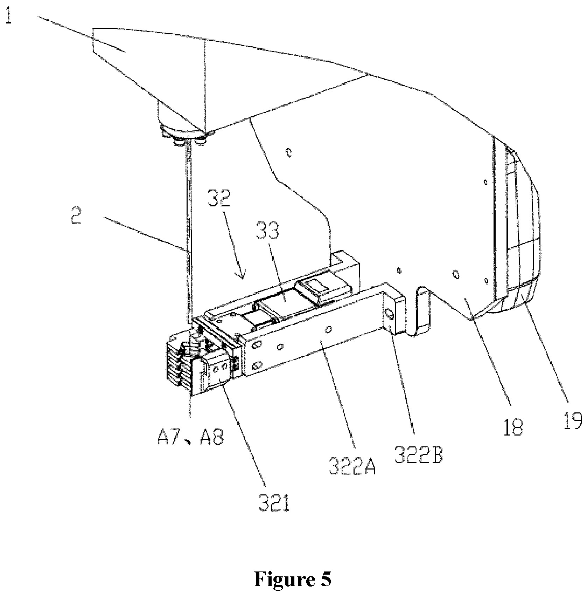

Figure 5 is a schematic structural diagram of a hand-eye system of the radiotherapy apparatus according to the second embodiment of the present disclosure. The hand-eye system includes a clamp 32 and an image acquisition device 19. The clamp 32 includes a griping portion 321 and two L-shaped support plates 322. The griping portion 321 is arranged under the beam needle 2 and is configured to grip the trocar 4. The griping portion 321 is fixed on the baseplate 18 of the handpiece through the two support plates 322. Each L-shaped support plate 322 includes a horizontal plate 322B and a vertical plate 322A. The horizontal plate 322B is fixed on the baseplate 18 of the handpiece. The two L-shaped support plates 322 are arranged parallel to each other with a gap and are perpendicular to the baseplate 18 of the handpiece. The clamp 32 is fixed on free ends of the two L-shaped support plates 322. An opening angle of the griping portion 321 is controlled by an impetus driving element 33 arranged between two vertical plates 322A. A gripping central axis of the griping portion 321 coincides with a central axis of the beam needle 2 and thus the central axis of the beam needle 2 coincides with a central axis of the trocar 4 when the clamp 32 grips the trocar 4 arranged on the body of the patient 300. In can be understood that in practice, it is difficult to achieve a complete coincidence between the gripping central axis of the clamp 32 and the central axis of the beam needle 2 and a deviation within a range is allowed.

It can be understood that a structure of the driving mechanism 100 is not limited to a structure described in the solution in the embodiment. The driving mechanism 100 may have other structure as long as the driving mechanism can accurately move within a three-dimensional space area relative to the patient 300.

The 3D image acquisition device 19 is mounted on a back surface of the baseplate 18 of the handpiece and is configured to acquire a 3D image of the trocar 4. The 3D image acquisition device 19 may be a 3D scanner configured to acquire a three-dimensional point cloud image of the trocar 4.

The clamp 32 and the 3D image acquisition device 19 form the mechanical hand-eye system of the radiotherapy apparatus. The hand-eye system is calibrated through a calibrator before being used. After the hand-eye system is calibrated, an image point cloud coordinate acquired by the 3D image acquisition device 19 may be converted into a coordinate based on a coordinate system of the driving mechanism 100 by the control terminal 200.

In therapy, medical care personnel control the trocar 4 to be inserted into the tumor lesion site of the patient 300. The 3D image acquisition device 19 scans to obtain a 3D point cloud image of the trocar 4. The control terminal 200 fits the 3D point cloud image of the trocar 4 to a circular tube shape and acquires a coordinate of the central axis of the trocar 4. The control terminal 200 controls the driving mechanism to move to cause the central axis of the clamp 32 to coincide with the central axis of the trocar 4 and to cause the clamp 32 to grip the trocar 4. After the clamp 32 grips the trocar 4, the control terminal 200 controls the handpiece 1 to move down relative to the baseplate so as to cause the beam needle 2 to insert into the trocar 4 and reach a predetermined position. The control terminal 200 controls the electron gun to start to emit a predetermined dose of electron beam. After being accelerated by the accelerating tube, the electron beam is shot into the body of the patient 300 through the beam needle 2, so as to ablate a diseased tissue.

It can be understood that the control terminal 200 may be directly mounted on the driving mechanism 100 as a whole. Alternatively, only a processor of the control terminal 200 is integrated with the driving mechanism 100 while a display screen and an input device of the control terminal 200 are arranged outside of the driving mechanism 100.

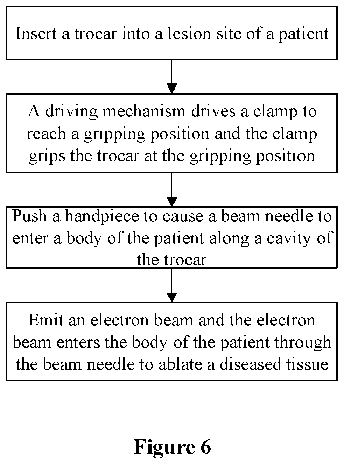

A therapy method using the radiotherapy apparatus according to the second embodiment is described below. Referring to Figure 6, the therapy method includes following steps (1) to (4).

In step (1), the trocar 4 is inserted into the lesion site of the patient 300.

In step (2), the driving mechanism 100 drives the clamp 32 to reach a gripping position. The clamp grips the trocar 4 at the gripping position. A 3D point cloud image of the trocar 4 is acquired based on a coordinate system of the driving mechanism 100. The gripping position at which the clamp 32 grips the trocar 4 is acquired based on the 3D point cloud image of the trocar 4.

In step (3), the handpiece 1 is pushed to cause the beam needle 2 to enter the body of the patient 300 through the trocar 4. The handpiece 1 may be pushed manually by medical care personnel. Alternatively, the handpiece 1 may be pushed automatically by the driving mechanism 100 after the clamp 32 grips the trocar 4.

In step (4), an electron beam is emitted and the emitted electron beam enters the body of the patient 300 through the beam needle 2 to ablate a diseased tissue.

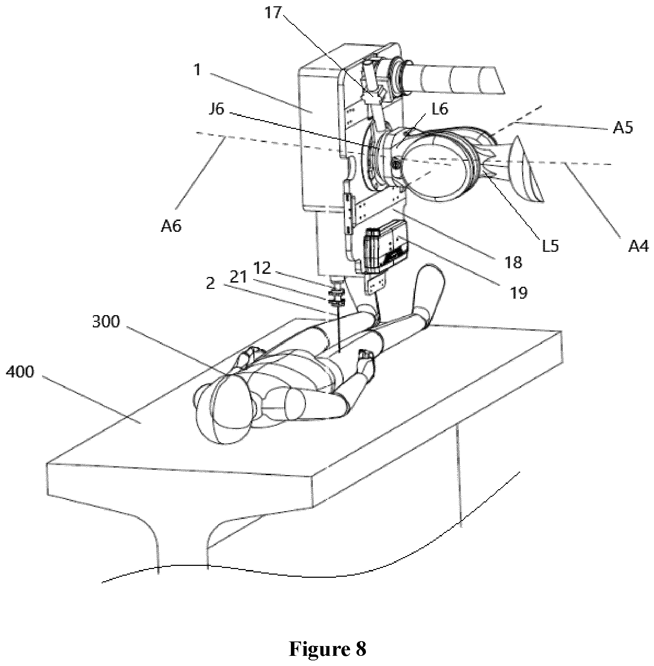

It can be understood that the radiotherapy apparatus according to the present disclosure is not limited to be used in combination with the trocar 4. Figure 7 is a schematic structural diagram of a radiotherapy apparatus according to a third embodiment of the present disclosure. Figure 8 is a schematic diagram showing a partial structure of the radiotherapy apparatus according to the third embodiment of the present disclosure. Compared with the second embodiment, referring to Figure 7 and Figure 8, the radiotherapy apparatus in the embodiment does not include the clamp 32.

In the embodiment, a visual positioning device acquires a 3D image of a body surface of the patient 300 corresponding to a tumor lesion site through the 3D scanner 19 and transmits the 3D image of the body surface of the patient to the control terminal 200. The control terminal 200 matches the acquired 3D image of the body surface of the patient 300 with a pre-stored tomographic image of the tumor lesion site of the patient 300, acquires a conversion between a coordinate system based on the tumor lesion site of the patient 300 and a coordinate system based on the driving mechanism 100, and controls the driving mechanism 100 to drive the beam needle 2 to move based on a predetermined therapy path planning so as to cause the beam needle 2 to be directly inserted into a predetermined position in the body of the patient 300. After the beam needle 2 reaches the predetermined position in the body of the patient 300, the control terminal 200 controls the electron gun to start to emit a predetermined dose of electron beam. After being accelerated by the accelerating tube, the electron beam is shot into the body of the patient 300 through the beam needle 2, so as to ablate a disease tissue.

A therapy method using the radiotherapy apparatus according to the third embodiment is described below. Referring to Figure 6, the therapy method includes following steps (1) to (3).

In step (1), a target position that the beam needle 2 is to reach is acquired. The target position may be outside the body of the patient 300 or inside the body of the patient 300. The target position may be acquired by: acquiring a lesion tomographic image of a lesion in the patient 300, acquiring a 3D point cloud image of a body surface corresponding to the lesion in the patient 300 based on a coordinate system of the driving mechanism 100, matching the 3D point cloud image of the body surface corresponding to the lesion in the patient 300 with the lesion tomographic image of the patient 300 and converting a coordinate of the lesion tomographic image of the patient 300 into a coordinate of the lesion tomographic image of the patient based on the coordinate system of the driving mechanism, and acquiring the target position that the beam needle 2 is to reach based on a predetermined therapy plan and the coordinate of the lesion tomographic image of the patient 300 based on the coordinate system of the driving mechanism 100.

In step (2), the driving mechanism 100 drives the beam needle 2 to reach the target position. The beam needle 2 enters the body of the patient 300. In a case that the target position is located above the lesion and outside the body of the patient 300, the beam needle 2 is manually pushed into the body of the patient 300 after reaching the target position. In a case that the target position is located inside the body of the patient 300, the driving mechanism 100 pushes the beam needle 2 into the body of the patient 300 to cause the beam needle 2 to directly reach the target position.

In step (3), an electron beam is emitted and the emitted electron beam enters the body of the patient 300 through the beam needle so as to ablate a diseased tissue.

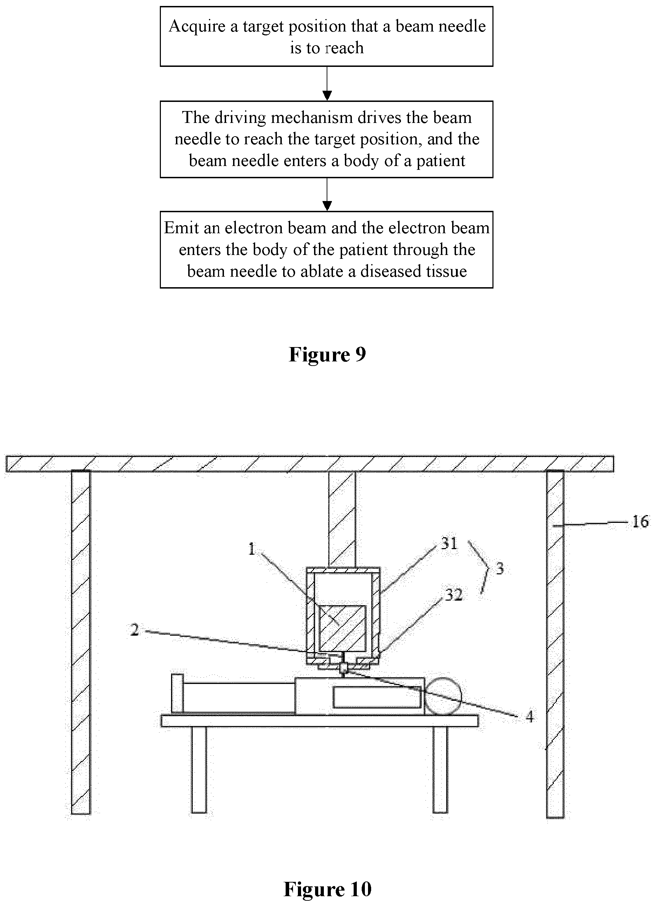

Figure 10 is a schematic structural diagram of the radiotherapy apparatus according to a fourth embodiment of the present disclosure. In order to cause the motion device 3 to rapidly dive the beam needle 2 to move, as shown in Figure 10, the motion device 3 includes a sliding rail 31 and a driving element (not shown in Figure 10). The sliding rail 31 is arranged along a direction along which the beam needle 2 moves. The accelerating tube 12 of the electron beam emitting device 1 is slidably arranged on the sliding rail 31. The driving element is arranged on the sliding rail 31 and is connected to the accelerating tube 12 to drive the accelerating tube 12 to slide in a straight line along the sliding rail 31. The accelerating tube 12 drives the beam needle 2 to move in a straight line when sliding. In practice, as shown in Figure 10, a fixed mount 16 may be arranged to fix the sliding rail 31 of the motion device 3. The patient 300 may lie flat or on his or her side relative to the motion device 3, as shown in Figure 10. The motion device 3 may be always located above the patient 300 with the movement of the motion device 3. In practice, the driving element may directly push the accelerating tube 12 using a cylinder. Alternatively, the driving element may drive the accelerating tube 12 to move up and down using a motor combined with a threaded spindle. When the driving element (not shown in Figure 10) drives the accelerating tube 12 to move towards the body, the accelerating tube 12 may rapidly drive, based on guidance quality of the sliding rail 31, the beam needle 2 to enter the tumor lesion site 8 in the body through the trocar 4 prearranged on the body. When the driving element drives the accelerating tube 12 to move away from the body, the beam needle 2 is drawn out of the trocar 4. In this way, the beam needle 2 can move rapidly.

In addition, as a preferable solution, the radiotherapy apparatus according to the embodiment further includes a clamp 32 arranged on the motion device 3. The clamp 32 is arranged on the baseplate 18 of the sliding rail 31 of the motion device 3 and is configured to grip the trocar 4 prearranged on the body. The trocar 4 is griped by the clamp 32 so that the trocar 4 does not interfere with the beam needle 2 due to shaking when the beam needle 2 is drawn into the body through the trocar 4.

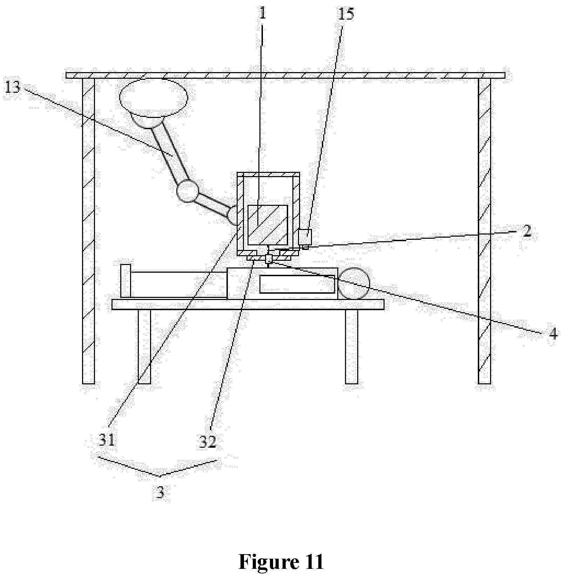

Figure 11 is a schematic structural diagram of a radiotherapy apparatus without a travelling crane according to a fifth embodiment of the present disclosure. Compared with the fourth embodiment, in the fifth embodiment, as shown in Figure 11, the sliding rail 31 of the motion device 3 is in fixed connection with a mechanical arm 13. The mechanical arm 13 may be configured to drive the whole motion device 3 to move within a predetermined area.

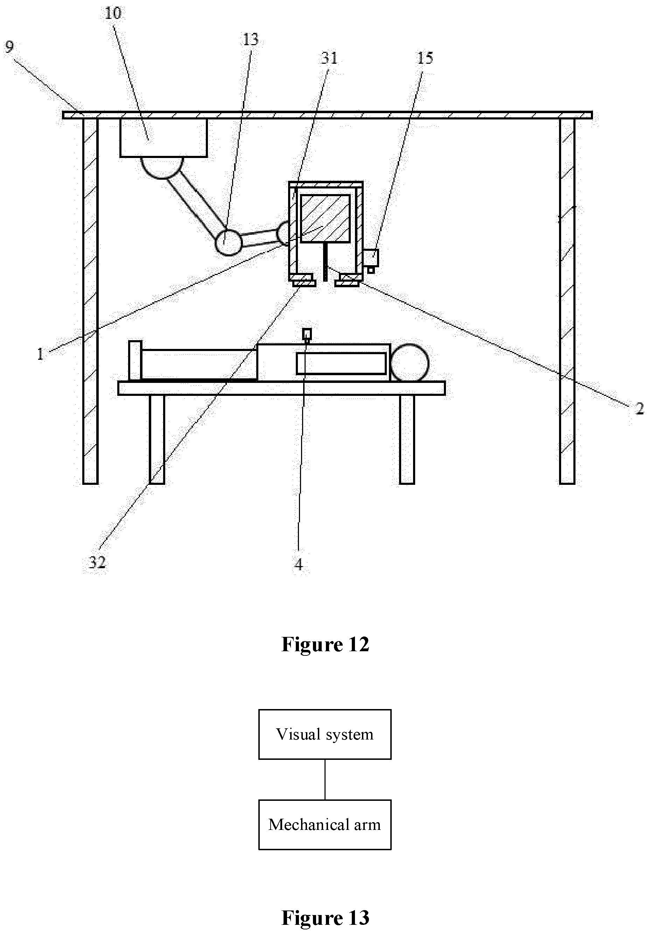

In addition, as a preferable solution, as shown in Figure 12, the radiotherapy apparatus according to the embodiment further includes a crossbeam 9 and a travelling crane 10. The crossbeam 9 is arranged relative to the body. Two ends of the crossbeam 9 are supported by supports. The travelling crane 10 is slidably arranged on the crossbeam 9 and is in a fixed connection with the mechanical arm 13 so as to drive the mechanical arm 13 to move along the crossbeam 9. A range of an area within which the motion device 3 moves can be extended through travelling of the travelling crane 10 along the crossbeam 9.

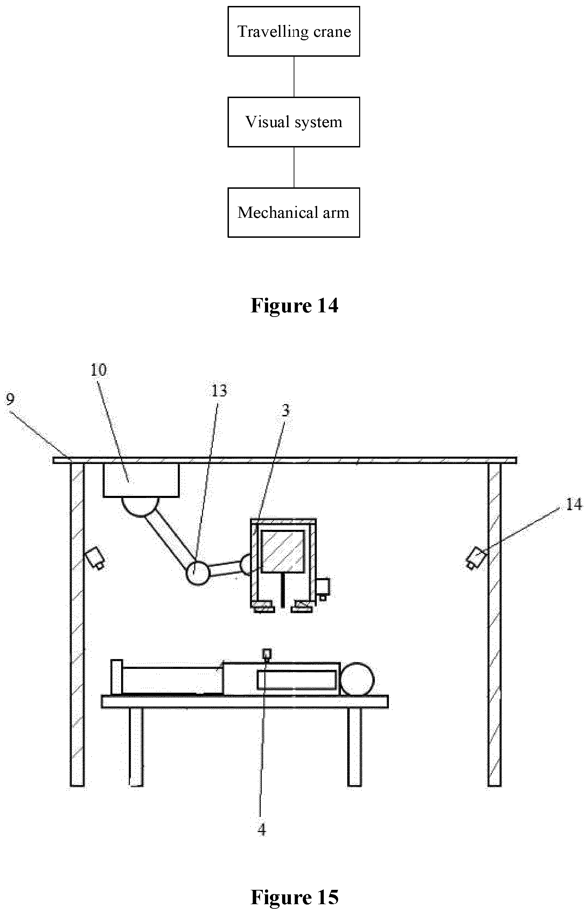

In addition, as shown in Figure 13 and Figure 14, the radiotherapy apparatus according to the embodiment further includes a visual system. The visual system is configured to acquire a central position of the trocar 4 and obtain an operation position of the motion devices 3 based on the acquired central position of the trocar 4. The visual system includes a first image shooting device 15 and a main control device communicating with the first image shooting device 15. The main control device is electrically connected to the mechanical arm 13 and is configured to control the mechanical arm 13 to move. In order to ensure that the image shooting device can accurately shoot the central position of the trocar 4 from top of the trocar 4, the image shooting device is preferably arranged on the sliding rail 31 of the motion device. In practice, the first image shooting device 15 may quire an image of a predetermined area and reports the image to the main control device. The main control device may be a computer, an industrial computer and the like. The main control device acquires color information of a central area of the trocar 4 from the image to calculate the central position of the trocar 4. The predetermined area in the embodiment is an area that the mechanical arm 13 can cover when moving. The trocar 4 prearranged on the body may be located at any position in the predetermined area. The main control device directly calculates the operation position of the motion device based on the central position and controls the mechanical arm 13 to drive the motion device 3 to move to the operation position. The clamp 32 grips the trocar 4. In addition, the beam needle 2 is coaxially arranged with the trocar 4 when the motion device 3 moves to the operation position to ensure that the beam needle 2 can be successfully drawn by the motion device 3 from the trocar 4 into the tumor lesion site in the body. As shown in Figure 6 and Figure 8, the main control device may be in communication connection with the travelling crane 10. The main control device drives the mechanical arm 13 by controlling the movement of the travelling crane 10 and causes the motion device 3 to move to the operation position by coordinating with the movement of the mechanical arm 13. In this way, the range of the area within which the motion device 3 moves is extended.

A sixth embodiment of the present disclosure relates to a radiotherapy apparatus. Compared with the second embodiment, in the embodiment, as shown in Figure 15, the radiotherapy apparatus in the embodiment further includes a second image shooting device 14. The second image shooting device 14 is configured to locate a predetermined position on the body at which the trocar 4 is arranged in advance and obtain a reference position of the motion device 3 based on the acquired predetermined position on the body at which the trocar 4 is arranged. Since the main control device calculates the position of the trocar 4 by acquiring color of the trocar 4, the second image shooting device 14 may be provided with a group of low resolution cameras. The cameras in the second image shooting device 14 are respectively arranged at two ends of the crossbeam 9 where each of the two ends contacts with a corresponding support. The cameras shoot an image of the trocar 4 and the image has less pixels. The main control device acquires the image and analyzes the position of the trocar 4 only based on the color of the trocar 4. The analyzed position serves as the reference position. The main control device controls the mechanical arm 13 to drive the motion device 3 to move to the analyzed position based on the analyzed position. Alternatively, the main control device controls the travelling crane 10 to drive the mechanical arm 13 to move to a position and then the main control device controls the mechanical arm 13 to move. In the above process, the main control device is only required to analyze an image with low resolution and determine the reference position of the trocar based on the color rather than analyze the reference position and the center of the trocar 4 simultaneously. Therefore, calculation and analysis performed on the image are simple. Hardware requirements and computing requirements for the mater device are low. In this case, the analysis can be completed using a main control device with a high performance cost ratio, which reduces cost of the main control device.

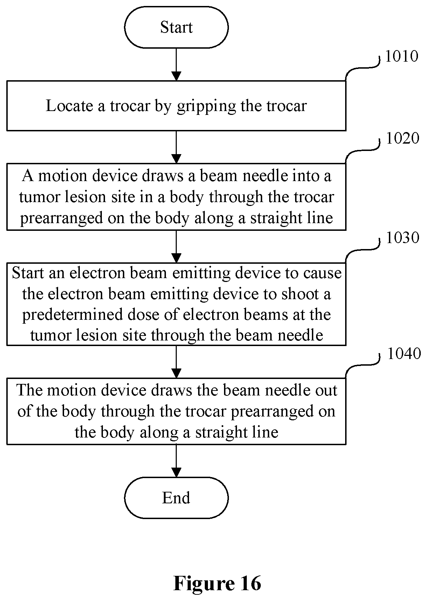

Figure 16 is a flowchart of a therapy method using anyone of the radiotherapy apparatuses according to the first, the fourth and the fifth embodiments of the present disclosure. The method includes steps 1020 to 1040.

In step 1020, the motion device draws the beam needle into the tumor lesion site of a tumor in a body along a straight line through the trocar prearranged on the body.

In step 1030, the electron beam emitting device starts to shoot a predetermined dose of electron beam at the tumor lesion site through the beam needle.

In step 1040, the motion device draws the beam needle out of the body along a straight line through the trocar prearranged on the body.

As can be seen from the above, the motion device drives the beam needle to move in a straight line so as to draw the beam needle into a tumour lesion site in the body through the trocar prearranged on the body. The electron beam emitting device emits an electron beam and the electron beam is shot at the tumour lesion site through the beam needle to ablate the tumour. In addition, after the electron beam is shot at the tumour lesion site, the beam needle may be drawn out of the body through the trocar under the drive of the motion device. In this way, shot deviation of the electron beam due to inaccurate positioning of the beam needle can be avoided. Furthermore, the electron beam has a weak penetrability. Therefore, not too much energy is accumulated in normal tissues in front of and behind the tumour lesion site, which avoids harm to normal cell tissues when the electron beam ablates the tumour, thereby reducing harm to the body.

The dose of electron beam emitted by the electron beam emitting device in the method is proportional to a size of the tumor in a predetermined manner. Energy of the electron beam ranges from 1MeV to 100MeV.

It should be noted that when the radiotherapy apparatus according to the embodiment is used to ablate a tumour, a therapeutic effect is different from that of radiotherapy apparatuses in conventional technologies and that of electron beam external irradiation using a conventional accelerator. For comparisons between the radiotherapy apparatus according to the embodiment and other methods for ablating a tumour, one may refer to Table 2.

**Table 2 Comparisons of the electron ablation knife and other methods for ablating a tumour**

| | Therapy principle | Whether normal tissues are passed through | Degree of wound | Degree of protection for normal tissues | Ray utilization |
|---|---|---|---|---|---|
| Electron ablation knife | Ionizing radiation | No | * | ***** | ***** |
| Surgery | Incision | Yes | ***** | *** | No |
| Argon-helium cryosurgery | Freezing | Yes | **** | *** | No |
| Intraoperative X-ray | Ionizing radiation | Yes | ***** | *** | *** |
| Intraoperative electron beam | Ionizing radiation | Yes | ***** | **** | **** |
| X-ray external irradiation | Ionizing radiation | Yes | *** | ** | ** |
| Electron beam external irradiation | Ionizing radiation | Yes | * * * | * * * | * * |
| Proton | Ionizing radiation | Yes | ** | * * * | * ** |
| Heavy ion | Ionizing radiation | Yes | ** | *** | **** |
| Focused ultrasound knife | Heating effect | Yes | *** | ** | No |
| Microwave radio frequency | Heating effect | Yes | **** | ** | No |
| Laser knife | Heating effect | Yes | *** | ** | No |

The intraoperative radiation therapy refers to that residual tumor cells or tissues are irradiated in the surgical wound area, which is usually used as a supplemental irradiation after surgery. In the electron beam external irradiation using a conventional accelerating tube, electron beams reach the tumor passing through normal tissues. Harm may be caused to normal tissues in a path along which the electron beams pass through. In addition, the electron beams have a limited irradiation depth. The surgical excision is very harmful to human body, which results difficulty in nursing. A needle used in the argon-helium cryosurgery is thick, which causes severe harm and great pain to patients. Because of existence of blood vessels and intestines, multiple needles are required to be inserted into a body of a patient, which is unbearable for the patient. A radio frequency needle is also thick, which causes difficulty in temperature control and heating effect wound that is prone to recur. In addition, blood vessels and intestines are required to be avoided. The laser ablation causes large wound, limited damage to the tumor and great pain to the patient.

Different from the other methods for ablating a tumour, with the method using the radiotherapy apparatus according to the embodiment, the accelerated and focused high brightness electron beam is directly transmitted to the tumor site through the threadlike beam needle so as to ablate the tumor. In this way, electrons can be directly accumulated at the tumor location site so as to directly ablate tumors in superficial and deep parts of the body. A therapy effect similar to that of surgical excision can be achieved after a single or multiple irradiations. The radiotherapy apparatus according to the embodiment can be used for accurate ablation of tumor, with few times of ablation, high single dose, and direct damage to tumor. In addition, electrons do not interact with normal tissues throughout the transmission path, which causes small energy loss. The electrons directly interact with the tumor, which leads to high ray utilization. In the radiotherapy apparatus according to the embodiment, a tube for transmitting the high brightness electron beam is very thin and a diameter of the tube is only a few millimeters. Therefore, a percutaneous puncture causes little harm to normal tissues. In addition to the above advantages, when the tumor is ablated using the above radiotherapy apparatus, a space occupancy is small. A machine room with a radiation protection function is not required and no gamma ray is generated.

As a preferable solution, in order to cause the beam needle to accurately and rapidly enter the body through the trocar, before the motion device draws the beam needle into the tumor lesion site in the body along a straight line through the trocar prearranged on the body, the method further includes step 1010. In step 1010, the trocar is located by griping the trocar.

It is easy to find from the above that this embodiment is a method embodiment corresponding to the first embodiment. This embodiment may be implemented in conjunction with the first embodiment. Relevant technical details mentioned in the first embodiment are still suitable for this embodiment. The relevant technical details are not repeated herein for reducing repetition. Accordingly, relevant technical details mentioned in this embodiment may also be applied to the first embodiment.

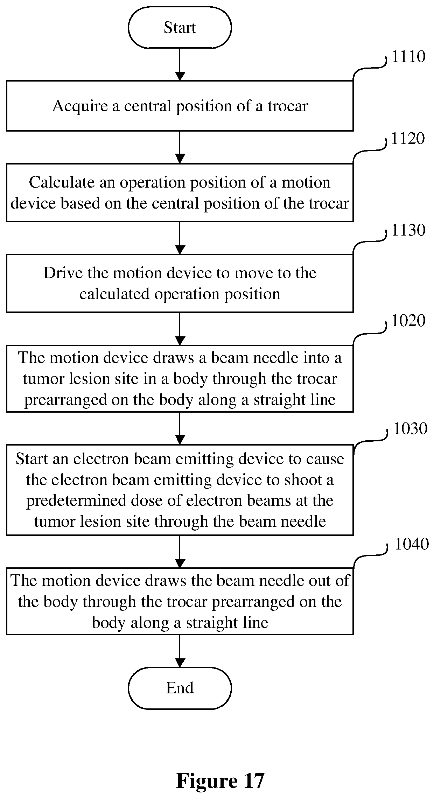

Figure 17 is a flowchart of a therapy method using anyone of the radiotherapy apparatuses according to the sixth and the seventh embodiments of the present disclosure. The method is improved based on the method using anyone of the radiotherapy apparatuses according to the first, the fourth and the fifth embodiments of the present disclosure. As shown in Figure 17, before the motion device draws the beam needle into the tumor lesion site in the body along a straight line through the trocar prearranged on the body, the improved method further includes steps 1110 to 1130.

In step 1110, a central position of the trocar is acquired.

The central position of the trocar may be acquired by acquiring, in a predetermined area, an image of the trocar arranged on the body. In the embodiment, the image in the predetermined area may be shot by the first image shooting device 15 as shown in Figure 12 and Figure 13. The predetermined area is an area within which the mechanical arm can cover when moving. It should be noted that before the image is shot, it is required to ensure that the trocar 4 prearranged on the body is located within the predetermined area and the trocar 4 may be located at any position within the predetermined area. In order to ensure that the image shooting device can accurately shoot the central position of the trocar 4 from top of the trocar 4, the shooting device is preferably arranged on the sliding rail 31 of the motion device 3.

The central position of the trocar 4 is obtained based on the acquired image. In the embodiment, a main control device such as a computer and an industrial computer may be used to acquire color information of the central area of the trocar from the image to calculate the central position of the trocar 4.

In step 1120, the operation position of the motion device is calculated based on the central position of the trocar.

After obtaining the central position of the trocar, the main control device may directly calculate the operation position of the motion device based on the central position.

In step 1130, the motion device is driven to move to the calculated operation position. The beam needle 2 is arranged coaxially with the trocar 4 when the motion device 3 moves to the operation position to ensure that the clamp 32 can accurately grip the trocar 4 and the beam needle 2 can be successfully drawn by the motion device 3 from the trocar 4 into the tumor lesion site in the body.

A mechanical arm 13 may be in fixed connection with the sliding rail 31 of the motion device 3 and may be electrically connected to the main control device so as to communicate with the main control device. Therefore, after calculating the operation position of the motion device 3, the main control device may directly control the mechanical arm 13 to drive the motion device to move to the operation position.

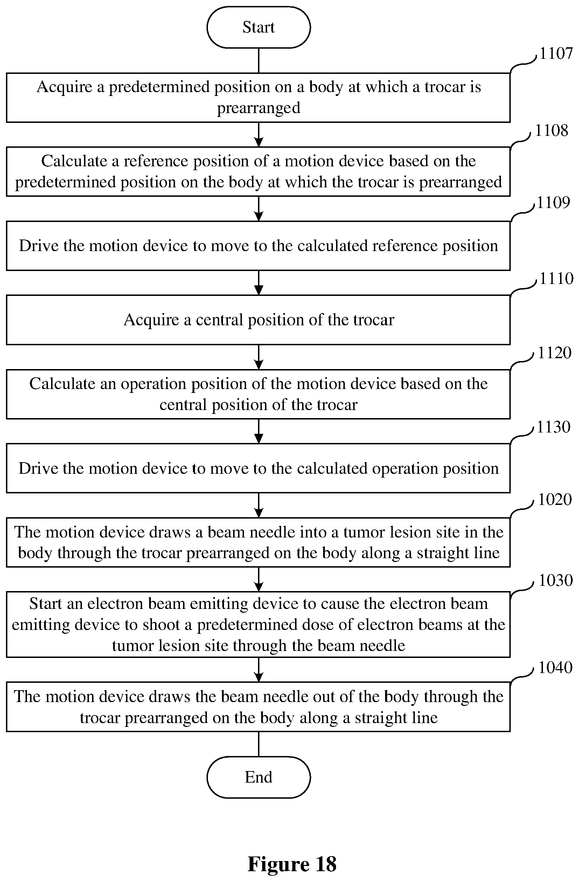

Figure 18 is a flowchart of another therapy method using anyone of the radiotherapy apparatuses according to the sixth and the seventh embodiments of the present disclosure. The another method is improved based on the therapy method using anyone of the radiotherapy apparatuses according to the sixth and the seventh embodiments of the present disclosure.

As shown in Figure 18, before step 1110, the improved method further includes steps 1107 to 1109.

In step 1107, a predetermined position on the body at which the trocar is prearranged is acquired. The predetermined position on the body at which the trocar is prearranged may be acquired by acquiring, in a predetermined area, an image of the trocar arranged on the body. In the embodiment, an additional image shooting device may be added to shoot the image in the predetermined area. A position at which the trocar is located is found in the image. In the embodiment, a main control device is used to acquire color information of the trocar from the image so as to calculate the predetermined position on the body at which the trocar is prearranged.

In step 1108, a reference position of the motion device is calculated based on the predetermined position on the body at which the trocar is prearranged. After acquiring the predetermined position on the body at which the trocar is prearranged, the main control device may directly calculate the reference position of the motion device based on the predetermined position.

In step 1109, the motion device is driven to move to the calculated reference position. The beam needle is located above the trocar when the motion device reaches the reference position. After calculating the reference position of the motion device, the main control device may directly control the mechanical arm to drive the motion device to move to the reference position. Alternatively, the mater device may control the travelling crane to drive the mechanical arm and then control the mechanical arm to move.

It can be seen from the above that the radiotherapy apparatus in the embodiment further includes an additional image shooting device. The additional image shooting device may be configured to locate a predetermined position on the body at which the trocar is arranged in advance. Since the main control device calculates the position of the trocar by acquiring color of the trocar, the video device may be provided with a group of low resolution cameras to shoot an image of the trocar. The image has less pixels. The main control device acquires the image and analyzes the position of the trocar only based on the color of the trocar. The analyzed position serves as a reference position. The main control device controls the mechanical arm to drive the motion device to move to the analyzed position. In the above process, the main control device is only required to analyze an image with less pixels and determine the reference position of the trocar based on the color rather than analyze the reference position and the center of the trocar simultaneously. Therefore, calculation and analysis performed on the image are simple. Hardware requirements and computing requirements for the mater device are low. In this case, the analysis can be completed using a main control device with a high performance cost ratio, which reduces cost of the main control device.

It is easy to find from the above that this embodiment is a method embodiment corresponding to the third embodiment. This embodiment may be implemented in conjunction with the third embodiment. Relevant technical details mentioned in the third embodiment are still suitable for this embodiment. The relevant technical details are not repeated herein for reducing repetition. Accordingly, relevant technical details mentioned in this embodiment may also be applied to the third embodiment.

Those skilled in the art may understand that the above embodiments are specific embodiments of the present disclosure, and in practice, various variations may be made in form and detail based on the above embodiments without deviating the scope of the present disclosure.

## Claims

1. A radiotherapy apparatus comprising:
a handpiece (1) provided with an electron accelerator and a beam needle (2), wherein the electron accelerator is configured to generate and accelerate an electron beam, the beam needle (2) is connected to the electron accelerator and is configured to cause the electron beam generated and accelerated by the electron accelerator to be shot into a body through the beam needle (2) to ablate a diseased tissue;
a trocar (4) on a body of a patient;
a driving mechanism (100) connected to the electron accelerator;
a processor configured to acquire a target position of the beam needle (2) based on a predetermined therapy plan; wherein
the driving mechanism (100) is further configured to drive the beam needle (2) to move to the target position in response to an instruction sent by the processor,
the handpiece (1) is connected to the driving mechanism (100) through a baseplate, a sliding structure is arranged between the handpice and the baseplate, and the handpice is able to move up and down along a direction along which the beam needle (2) extends, and
the radiotherapy apparatus further comprises a mechanical hand-eye system, wherein the mechanical hand-eye system comprises a clamp (32) and a 3D scanner (19) mounted on the baseplate, and wherein the 3D scanner (19) is configured to scan the trocar (4) on the body of the patient to obtain a three-dimensional point cloud image of the trocar (4), and the processor is configured to acquire a position at which the clamp (32) grips the trocar (4) based on information of the point cloud image of the trocar (4).

2. The radiotherapy apparatus according to claim 1, wherein a gripping central axis of the clamp (32) coincides with a central axis of the beam needle (2).

3. The radiotherapy apparatus according to claim 1 or 2, wherein the driving mechanism (100) is a driving mechanism of a six-axis mechanical arm.

4. A radiotherapy apparatus comprising:
an electron beam emitting device configured to emit an electron beam;
a beam needle (2) connected to the electron beam emitting device and configured to shoot the electron beam emitted by the electron beam emitting device at a tumor lesion site of a tumor in a body to ablate the tumor;
a trocar (4) prearranged on the body;
a motion device configured to drive the beam needle (2) to move in a straight line, to draw the beam needle (2) into the tumor lesion site in the body through the trocar (4) prearranged on the body and to draw the beam needle (2) out of the body through the trocar (4) prearranged on the body after the beam needle (2) shoots the electron beam at the tumor lesion site, and
a clamp (32), wherein the clamp (32) is arranged on the motion device and is configured to grip or loosen the trocar (4);
wherein
a mounting support is provided at an end of the motion device, wherein: the mounting support is provided with a sliding rail and a driving element; the sliding rail is arranged along a direction along which the beam needle (2) moves; the electron beam emitting device is slidably arranged on the sliding rail; the driving element is arranged on the sliding rail and is connected to the electron beam emitting device and is configured to drive the electron beam emitting device to slide along the sliding rail; and
the electron beam emitting device is configured to drive the beam needle (2) to move in a straight line when the electron beam emitting device slides.

5. The radiotherapy apparatus according to claim 4, wherein the electron beam emitting device comprises:
an electron gun configured to emit an electron beam;
an accelerating tube connected to the beam needle (2) and configured to receive the electron beam emitted by the electron gun and to accelerate the electron beam.

6. The radiotherapy apparatus according to claim 5, wherein
two ends of the accelerating tube are respectively an inlet for receiving the electron beam emitted by the electron gun and an outlet for shooting the electron beam into to the beam needle (2); and
the accelerating tube is further provided with a microwave inlet for inputting microwave to the accelerating tube.

## Patentansprüche

1. Strahlentherapiegerät mit:
einem Handstück (1), das mit einem Elektronenbeschleuniger und einer Strahlnadel (2) versehen ist, wobei der Elektronenbeschleuniger konfiguriert ist, um einen Elektronenstrahl zu erzeugen und zu beschleunigen, und die Strahlnadel (2) mit dem Elektronenbeschleuniger verbunden ist und konfiguriert ist, um zu bewirken, dass der vom Elektronenbeschleuniger erzeugte und beschleunigte Elektronenstrahl durch die Strahlnadel (2) in einen Körper geschossen wird, um krankes Gewebe abzutragen;
einem Trokar (4) an einem Körper eines Patienten;
einem mit dem Elektronenbeschleuniger verbundenen Antriebsmechanismus (100);
einem Prozessor, der konfiguriert ist, um auf der Grundlage eines vorgegebenen Therapieplans eine Zielposition der Strahlnadel (2) zu erfassen; wobei
der Antriebsmechanismus (100) ferner konfiguriert ist, um in Reaktion auf eine vom Prozessor gesendete Anweisung die Strahlnadel (2) so anzutreiben, dass sie sich in die Zielposition bewegt,
das Handstück (1) mit dem Antriebsmechanismus (100) über eine Grundplatte verbunden ist, eine Gleitstruktur zwischen dem Handstück und der Grundplatte angeordnet ist und das Handstück entlang einer Richtung, in der sich die Strahlennadel (2) erstreckt, auf und ab beweglich ist, und
die Strahlentherapievorrichtung ferner ein mechanisches Hand-Auge-System umfasst, wobei das mechanische Hand-Auge-System eine Klemme (32) und einen auf der Grundplatte montierten 3D-Scanner (19) umfasst, und wobei der 3D-Scanner (19) konfiguriert ist, um den Trokar (4) am Körper des Patienten zu scannen, um ein dreidimensionales Punktwolkenbild des Trokars (4) zu erhalten, und der Prozessor konfiguriert ist, um basierend auf Informationen des Punktwolkenbildes des Trokars (4) eine Position zu erfassen, an der die Klemme (32) den Trokar (4) greift.

2. Strahlentherapiegerät nach Anspruch 1, bei dem eine Greifmittelachse der Klemme (32) mit einer Mittelachse der Strahlnadel (2) übereinstimmt.

3. Strahlentherapiegerät nach Anspruch 1 oder 2, bei dem der Antriebsmechanismus (100) ein Antriebsmechanismus eines sechsachsigen mechanischen Arms ist.

4. Strahlentherapiegerät mit:
einer Elektronenstrahl-Emissionsvorrichtung, die konfiguriert ist, um einen Elektronenstrahl zu emittieren;
einer Strahlnadel (2), die mit der Elektronenstrahl-Emissionsvorrichtung verbunden ist und konfiguriert ist, um den von der Elektronenstrahl-Emissionsvorrichtung emittierten Elektronenstrahl auf eine Tumorläsionsstelle eines Tumors in einem Körper zu schießen, um den Tumor abzutragen;
einem Trokar (4), der vorab am Körper angeordnet ist;
einer Bewegungsvorrichtung, die konfiguriert ist, um die Strahlnadel (2) zu einer Bewegung in einer geraden Linie anzutreiben, um die Strahlnadel (2) durch den am Körper vorab angeordneten Trokar (4) in die Tumorläsionsstelle im Körper einzuführen und um die Strahlnadel (2) durch den am Körper vorab angeordneten Trokar (4) aus dem Körper herauszuziehen, nachdem die Strahlnadel (2) den Elektronenstrahl auf die Tumorläsionsstelle geschossen hat, und
einer Klemme (32), wobei die Klemme (32) an der Bewegungsvorrichtung angeordnet ist und konfiguriert ist, um den Trokar (4) zu greifen oder loszulassen; wobei
eine Befestigungshalterung an einem Ende der Bewegungsvorrichtung vorgesehen ist, wobei: die Befestigungshalterung mit einer Gleitschiene und einem Antriebselement versehen ist; die Gleitschiene entlang einer Richtung angeordnet ist, entlang der sich die Strahlnadel (2) bewegt; die Elektronenstrahl-Emissionsvorrichtung verschiebbar auf der Gleitschiene angeordnet ist; das Antriebselement auf der Gleitschiene angeordnet und mit der Elektronenstrahl-Emissionsvorrichtung verbunden ist und konfiguriert ist, um die Elektronenstrahl-Emissionsvorrichtung zum Gleiten entlang der Gleitschiene anzutreiben; und
die Elektronenstrahl-Emissionsvorrichtung konfiguriert ist, um die Strahlnadel (2) zum Bewegen in einer geraden Linie anzutreiben, wenn die Elektronenstrahl-Emissionsvorrichtung gleitet.

5. Strahlentherapiegerät nach Anspruch 4, wobei die Elektronenstrahl-Emissionsvorrichtung umfasst:
eine Elektronenkanone, die konfiguriert ist, um einen Elektronenstrahl zu emittieren;
eine Beschleunigungsröhre, die mit der Strahlnadel (2) verbunden ist und konfiguriert ist, um den von der Elektronenkanone emittierten Elektronenstrahl zu empfangen und den Elektronenstrahl zu beschleunigen.

6. Strahlentherapiegerät nach Anspruch 5, bei dem die beiden Enden der Beschleunigungsröhre jeweils ein Einlass zum Empfangen des von der Elektronenkanone emittierten Elektronenstrahls und ein Auslass zum Abschießen des Elektronenstrahls in die Strahlnadel (2) sind; und
die Beschleunigungsröhre ferner mit einem Mikrowelleneinlass zum Einspeisen von Mikrowellen in die Beschleunigungsröhre versehen ist.

## Revendications

1. Appareil de radiothérapie, comprenant :
une pièce à main (1) équipée d'un accélérateur d'électrons et d'une aiguille de faisceau (2), dans lequel l'accélérateur d'électrons est configuré pour générer et accélérer un faisceau d'électrons, l'aiguille de faisceau (2) est connectée à l'accélérateur d'électrons et est configurée pour amener le faisceau d'électrons généré et accéléré par l'accélérateur d'électrons à être tiré dans un corps à travers l'aiguille de faisceau (2) pour ablater un tissu malade ;
un trocart (4) sur le corps d'un patient ;
un mécanisme d'entraînement (100) connecté à l'accélérateur d'électrons ;
un processeur configuré pour acquérir une position cible de l'aiguille de faisceau (2) sur la base d'un plan de traitement prédéterminé ; dans lequel
le mécanisme d'entraînement (100) est en outre configuré pour entraîner l'aiguille de faisceau (2) à se déplacer vers la position cible en réponse à une instruction envoyée par le processeur,
la pièce à main (1) est connectée au mécanisme d'entraînement (100) par l'intermédiaire d'une plaque de base, une structure coulissante est agencée entre la pièce à main et la plaque de base, et la pièce à main est capable de se déplacer vers le haut et vers le bas le long d'une direction le long de laquelle s'étend l'aiguille de faisceau (2), et
l'appareil de radiothérapie comprend en outre un système main-œil mécanique, dans lequel le système main-œil mécanique comprend une pince (32) et un scanner 3D (19) monté sur la plaque de base, et dans lequel le scanner 3D (19) est configuré pour balayer le trocart (4) sur le corps du patient afin d'obtenir une image de nuage de points en trois dimensions du trocart (4), et le processeur est configuré pour acquérir une position au niveau de laquelle la pince (32) saisit le trocart (4) sur la base des informations de l'image de nuage de points du trocart (4).

2. Appareil de radiothérapie selon la revendication 1, dans lequel un axe central de préhension de la pince (32) coïncide avec un axe central de l'aiguille de faisceau (2).

3. Appareil de radiothérapie selon la revendication 1 ou 2, dans lequel le mécanisme d'entraînement (100) est un mécanisme d'entraînement d'un bras mécanique à six axes.

4. Appareil de radiothérapie, comprenant :
un dispositif d'émission de faisceau d'électrons configuré pour émettre un faisceau d'électrons ;
une aiguille de faisceau (2) connectée au dispositif d'émission de faisceau d'électrons et configurée pour tirer le faisceau d'électrons émis par le dispositif d'émission de faisceau d'électrons au niveau d'un site de lésion tumorale d'une tumeur dans un corps pour ablater la tumeur ;
un trocart (4) pré-agencé sur le corps ;
un dispositif de mouvement configuré pour entraîner l'aiguille de faisceau (2) à se déplacer en ligne droite, pour tirer l'aiguille de faisceau (2) jusque dans le site de la lésion tumorale dans le corps à travers le trocart (4) pré-agencé sur le corps et pour tirer l'aiguille de faisceau (2) hors du corps à travers le trocart (4) pré-agencé sur le corps après que l'aiguille de faisceau (2) ait tiré le faisceau d'électrons au niveau du site de lésion tumorale, et
une pince (32), la pince (32) étant agencée sur le dispositif de mouvement et étant configurée pour saisir ou libérer le trocart (4) ;
dans lequel :
un support de montage est prévu à une extrémité du dispositif de mouvement, dans lequel : le support de montage est pourvu d'un rail coulissant et d'un élément d'entraînement ; le rail coulissant est agencé le long d'une direction le long de laquelle l'aiguille de faisceau (2) se déplace ; le dispositif d'émission de faisceau d'électrons est agencé de manière coulissante sur le rail coulissant ; l'élément d'entraînement est agencé sur le rail coulissant et est connecté au dispositif d'émission de faisceau d'électrons et est configuré pour entraîner le dispositif d'émission de faisceau d'électrons à coulisser le long du rail coulissant ; et
le dispositif d'émission de faisceau d'électrons est configuré pour entraîner l'aiguille de faisceau (2) à se déplacer en ligne droite lorsque le dispositif d'émission de faisceau d'électrons coulisse.

5. Appareil de radiothérapie selon la revendication 4, dans lequel le dispositif d'émission de faisceau d'électrons comprend :
un canon à électrons configuré pour émettre un faisceau d'électrons ;
un tube d'accélération connecté à l'aiguille de faisceau (2) et configuré pour recevoir le faisceau d'électrons émis par le canon à électrons et pour accélérer le faisceau d'électrons.

6. Appareil de radiothérapie selon la revendication 5, dans lequel
deux extrémités du tube d'accélération sont respectivement une entrée pour recevoir le faisceau d'électrons émis par le canon à électrons et une sortie pour tirer le faisceau d'électrons dans l'aiguille de faisceau (2) ; et
le tube d'accélération est en outre pourvu d'une entrée de micro-ondes pour entrer des micro-ondes dans le tube d'accélération.
